# FASCICULE DE BREVET EUROPEEN

(11) **EP 4 011 405 B1**
(45) Date de publication et mention de la délivrance du brevet: **08.01.2025**
(21) Numéro de dépôt: 21208582.3
(22) Date de dépôt: 16.11.2021
(51) Int. Cl.: A61L 2/18, A61L 2/20, C12G 3/07, B08B 9/08, C12H 1/22, B27K 5/00, B27K 3/02, B27K 3/16

(54) **PROCÉDÉ DE NETTOYAGE D'UN FÛT EN BOIS**
VERFAHREN ZUR REINIGUNG EINES HOLZFASSES
METHOD FOR CLEANING A WOODEN BARREL

(30) Priorité: 08.12.2020 FR 2012820
(43) Date de publication de la demande: 15.06.2022
(73) Titulaire: Tonnellerie de l'Entre-Deux-Mers (T.E.D.E.M), 33360 Lignan-de-Bordeaux (FR)
(72) Inventeur: DESAGES, Thibault, 33360 LIGNAN-DE-BORDEAUX (FR)
(74) Mandataire: LLR

(56) Documents cités:
- CN-A- 109 568 620
- US-A1- 2004 245 281
- US-A1- 2014 065 013
- US-A1- 2017 283 287

## Description

### DOMAINE TECHNIQUE DE L'INVENTION

L'invention concerne un procédé de nettoyage d'un fût en bois, tel qu'un tonneau ou une barrique, utilisé pour l'élevage de vin permettant de réutiliser le fût pour, par exemple, un nouvel élevage de vin, un vieillissement de boisson alcoolisée ou un transport de boisson alcoolisée.

### ARRIÈRE-PLAN TECHNIQUE DE L'INVENTION

L'élevage dans un fût est destiné, à l'aide de réactions physico-chimiques, à obtenir par exemple du vin, un spiritueux, une bière ou du vinaigre. De manière connue, le bois des douelles présente des pores, par lesquels le liquide pénètre selon une certaine épaisseur dans les douelles durant l'élevage. À l'inverse, l'air passe dans le liquide par ces mêmes pores, lui apportant une micro-oxygénation.

Le liquide infiltré entre les douelles, ou dans les douelles, peut augmenter les risques de contaminations microbiologiques notamment à cause des éléments à base de soufre utilisés pendant l'élevage et pouvant être responsables de défauts du produit obtenu. C'est pourquoi, après un processus d'élevage notamment de vin rouge, les fûts sont généralement revendus pour être remplacés par des neufs. À cause de ses infiltrations, un tonneau est revendu de dix à vingt fois moins cher qu'un neuf. Pourtant, le bois de chêne des tonneaux n'est en général pas structurellement altéré.

Il est connu de raboter ou sabler la face interne des douelles et des fonds d'un tonneau afin de mécaniquement retirer une épaisseur au moins équivalente à l'épaisseur d'infiltration du vin. Toutefois, cette diminution d'épaisseur augmente le risque de fuite et casse les fibres du bois au point de dégrader la résistance mécanique du tonneau. En effet, la résistance mécanique des douelles est importante, leur agencement en cercle permettant notamment de répartir la force exercée sur chacune des autres douelles. Le tonneau peut ainsi être stocké, roulé, basculé, vide ou plein, sans risque de casse.

Par ailleurs, d'autres solutions sans enlèvement de matière typiquement par circulation d'eau dans le fût existent, mais ne permettent pas comme le rabotage ou le sablage de retirer tous les résidus infiltrés en profondeur en particulier ceux à base de soufre. On connaît les documents US 2017/283287 A1, US 2004/245281 A1, US 2014/065013 A1 et CN 109 568 620 A.

### RÉSUMÉ DE L'INVENTION

L'invention a notamment pour but de fournir un nouveau procédé de nettoyage d'un fût en bois permettant notamment de retirer tout résidu infiltré dans les fibres du bois tel que les contaminations microbiologiques induites pendant un processus d'élevage d'une boisson alcoolisée ou bien tel que les pigments de vin rouge en redonnant au fût un aspect et des propriétés intrinsèques similaires ou identiques à un état neuf sans détériorer les fibres de bois.

À cet effet, l'invention se rapporte à un procédé de nettoyage d'un fût en bois **caractérisé** en ce qu'il comporte une étape de désinfection de l'intérieur du fût comprenant au moins une phase d'introduction de peroxyde d'hydrogène à l'intérieur du fût et une phase d'introduction de vapeur d'eau dans le fût avant ou après la phase d'introduction de peroxyde d'hydrogène à l'intérieur du fût afin d'aseptiser le bois du fût en profondeur et de retirer tout résidu infiltré dans les fibres du bois tel que des pigments.

Avantageusement selon l'invention, le pouvoir oxydant du peroxyde d'hydrogène permet de diminuer à une quantité négligeable voire indétectable les contaminations microbiologiques et notamment tout élément à base de soufre sans rendre plus sensible à l'oxydation le bois du fût. En outre, toute coloration du bois, occasionnée lors l'élevage d'une boisson alcoolisée dans le fût, est retirée permettant de retrouver un aspect neutre du bois sur tout le fût. Enfin, la surface interne du fût est, avantageusement selon l'invention, entièrement désinfectée en éliminant notamment toute acidité volatile ou de surface. Le fût est donc rendu totalement neutre par rapport à l'élevage de la boisson alcoolique pour lequel il a pu être utilisé ce qui lui permet de pouvoir être réutilisé notamment pour un nouvel élevage de boisson alcoolique dans les mêmes conditions qu'un fût neuf.

La phase d'introduction de vapeur d'eau permet de chauffer et ramollir les fibres du bois pour ouvrir suffisamment les pores du bois afin de faire pénétrer le peroxyde d'hydrogène dans le bois selon une épaisseur suffisante à enlever tout résidu qui y est incrusté. En outre, la phase d'introduction de vapeur d'eau permet d'amener le peroxyde d'hydrogène à une température où son efficacité d'aseptisation est optimale.

L'invention peut également comporter l'une ou plusieurs des caractéristiques optionnelles suivantes, prises seules ou en combinaison.

Lors de la phase d'introduction de peroxyde d'hydrogène à l'intérieur du fût, une solution de peroxyde d'hydrogène à 35 % mol est insérée dans le fût selon une proportion de 0,5 ml à 3,5 ml par litre de volume intérieur de fût et, préférentiellement, selon une proportion de 1,0 ml à 2,3 ml par litre de volume intérieur de fût.

La phase d'introduction de vapeur d'eau dans le fût est utilisée, préférentiellement, pour amener le peroxyde d'hydrogène à l'intérieur du fût à une température comprise entre 60°C et 80°C.

L'étape de désinfection comporte une phase de fermeture du fût après la phase d'introduction de vapeur d'eau dans le fût pour, en refroidissant, créer une dépression interne et refermer progressivement les pores du bois afin d'améliorer l'évacuation de tout résidu infiltré dans les fibres du bois. On comprend que cette phase de fermeture permet notamment de faire diminuer progressivement la température du volume intérieur du fût fermé pour que la dépression favorise un flux d'air au travers des pores du bois de la face externe vers la face interne et la contraction des pores du bois va mécaniquement expulser tout résidu présent dans les pores vers le volume intérieur du fût 1 sans que les résidus extraits ne puissent s'y incruster à nouveau. La durée de la phase de fermeture peut ainsi être comprise entre une heure et quatre heures.

La phase de fermeture du fût est maintenue pendant au moins une durée prédéterminée préférentiellement dépendante de la température à laquelle le fût a été chauffé pendant la phase d'introduction de la vapeur d'eau. Si la différence de température par rapport à la température ambiante est très importante, il pourra être nécessaire de prévoir un dispositif de compensation de pression comme une soupape pour éviter que la dépression à l'intérieur du fût créée par le refroidissement induise la détérioration des fonds du fût.

Lors de l'étape de désinfection, le fût peut être déplacé sur lui-même pour améliorer l'homogénéisation de l'aseptisation de l'intérieur du fût. Typiquement, le déplacement sur lui-même peut être envisagé après la phase d'introduction de peroxyde d'hydrogène à l'intérieur du fût et/ou lors de la phase de fermeture du fût.

Préférentiellement, l'étape de désinfection comporte une phase finale de lavage de l'intérieur du fût afin de rincer le bois de l'intérieur du fût. Cette phase peut également être utilisée pour détartrer l'intérieur du fût.

En outre, suivant l'état de saleté du fût, une étape de prélavage de l'intérieur du fût avant l'étape de désinfection peut être préférable afin de retirer toute matière organique en excès déposée sur la surface interne du fût.

### BRÈVE DESCRIPTION DES DESSINS

D'autres particularités et avantages de l'invention ressortiront clairement de la description qui en est faite ci-après, à titre indicatif et nullement limitatif, en référence aux dessins annexés, dans lesquels :
- la figure 1 est une vue schématique d'un fût pour l'élevage d'une boisson alcoolisée ;
- la figure 2 est une vue partielle d'une douelle après un élevage de vin rouge ;
- la figure 3 est un diagramme fonctionnel du procédé selon l'invention.

### DESCRIPTION DÉTAILLÉE D'AU MOINS UN MODE DE RÉALISATION DE L'INVENTION

Sur les différentes figures, les éléments identiques ou similaires portent les mêmes références, éventuellement additionnés d'un indice. La description de leur structure et de leur fonction n'est donc pas systématiquement reprise.

Dans tout ce qui suit, les orientations sont les orientations des figures. En particulier, les termes « supérieur », « inférieur », « gauche », « droit », « au-dessus », « en-dessous », « vers l'avant » et « vers l'arrière » s'entendent généralement par rapport au sens de représentation des figures.

Par « fût 1 », on entend tous les types de contenant en bois, tel qu'un tonneau ou une barrique, destiné à l'élevage, au vieillissement ou au transport de boissons alcoolisées comme par exemple du vin, du cidre, de l'eau-de-vie, de la bière ou du vinaigre.

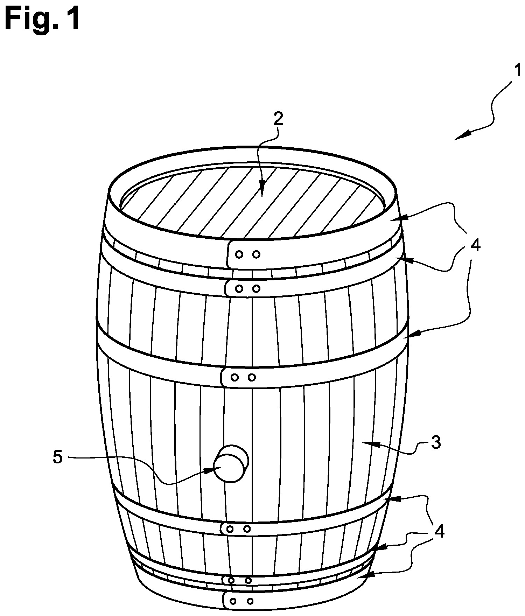

Un exemple de fût 1 en bois, comme par exemple du chêne, est illustré à la figure 1. Le fût 1 comporte de manière connue une partie sensiblement cylindrique formée par des douelles orientées verticalement et plaquées les unes contre les autres par des cercles 4. Le volume sensiblement cylindrique est fermé en haut et en bas par des fonds 2 (un seul visible à la figure 1). Enfin, une des douelles 3, dite douelle maîtresse, comporte un trou de bonde qui est bouché de manière hermétique avec une bonde 5 en silicone.

L'élevage dans un fût 1 en bois, comme par exemple un tonneau de 225 I, est destiné, à l'aide de réactions physico-chimiques, à obtenir par exemple du vin. De manière connue, le bois des douelles 3 présente des pores, par lesquels, sur la face interne 7, le liquide à élever pénètre selon une certaine épaisseur E dans les douelles 3 durant l'élevage. À l'inverse, par la face externe 6, l'air passe dans le liquide à élever par ces mêmes pores, lui apportant une micro-oxygénation.

Le liquide infiltré entre les douelles 3, ou dans les douelles 3, peut augmenter les risques de contaminations microbiologiques, responsables de défauts du produit élevé. C'est pourquoi, après un processus d'élevage notamment de vin rouge, les fûts 1 sont généralement revendus pour être remplacés par des neufs. À cause de ses infiltrations, un fût 1 est revendu de dix à vingt fois moins cher qu'un neuf. Pourtant, le bois par exemple de chêne des fûts 1 n'est en général pas structurellement altéré.

L'invention a notamment pour but de fournir un nouveau procédé 11 de nettoyage d'un fût 1 en bois permettant de retrouver une surface interne 7 neutre notamment quant à sa coloration et ses tanins afin de pouvoir l'utiliser à nouveau comme un fût 1 neuf. Avantageusement selon l'invention, le procédé 11 permet notamment de retirer tout résidu infiltré dans les fibres du bois y compris les éventuels pigments de vin rouge en redonnant au fût 1 un aspect et des propriétés intrinsèques similaires ou identiques à un état neuf sans détériorer les fibres de bois.

À cet effet, le procédé 11 de nettoyage d'un fût 1 en bois comporte une étape 13 de désinfection de l'intérieur du fût 1. Suivant l'état de saleté de la surface interne 7 du fût 1, le procédé 11 de nettoyage peut comporter une étape 21 de prélavage de l'intérieur du fût 1 avant l'étape 13 de désinfection afin de retirer toute matière organique en excès déposée sur la surface interne 7 du fût 1. L'étape 21 de prélavage peut être obtenue, par exemple, par la circulation de vapeur d'eau et/ou d'eau chaude dans le fût 1, c'est-à-dire l'introduction dans le fût 1 par une conduite d'entrée passant par le trou de bonde avec la libre possibilité de s'échapper du fût 1 par une sortie (qui peut être le trou de bonde orienté vers le bas), pendant une durée autour de cinq minutes. Bien entendu, suivant le degré de saleté de la surface interne 7 du fût, la durée de l'étape 21 de prélavage peut être plus longue ou plus courte que cinq minutes.

Avantageusement selon l'invention, l'étape 13 de désinfection comporte au moins une phase 15 d'introduction de peroxyde d'hydrogène (H₂O₂) à l'intérieur du fût 1 afin d'aseptiser le bois du fût 1 en profondeur. En effet, le demandeur a découvert que le pouvoir oxydant du peroxyde d'hydrogène permet de diminuer à une quantité négligeable voire indétectable tout élément à base de soufre sans rendre plus sensible à l'oxydation le bois du fût 1. En outre, toute coloration du bois, occasionnée lors l'élevage d'une boisson alcoolisée dans le fût 1, est retirée permettant de retrouver un aspect neutre du bois sur tout le fût 1. Enfin, la surface interne 7 du fût 1 est, avantageusement selon l'invention, entièrement désinfectée en éliminant notamment toute acidité volatile ou de surface. Le fût 1 est donc rendu totalement neutre par rapport à l'élevage de la boisson alcoolique pour lequel il a pu être utilisé ce qui lui permet de pouvoir être réutilisé notamment pour un nouvel élevage de boisson alcoolique dans des conditions similaires à un fût 1 neuf.

Lors de la phase d'introduction de peroxyde d'hydrogène à l'intérieur du fût 1, une solution de peroxyde d'hydrogène à 35 % mol est insérée dans le fût 1 par le trou de bonde selon une proportion de 0,5 ml à 3,5 ml par litre de volume intérieur de fût 1 et, préférentiellement, selon une proportion de 1,0 ml à 2,3 ml par litre de volume intérieur de fût 1, c'est-à-dire notamment 1,0 ml, 1,1 ml, 1,2 ml, 1,3 ml, 1,4 ml, 1,5 ml, 1,6 ml, 1,7 ml, 1,8 ml, 1,9 ml, 2,0 ml, 2,1 ml, 2,2 ml et 2,3 ml par litre de volume intérieur de fût 1.

Bien évidemment, suivant la concentration de la solution de peroxyde d'hydrogène, c'est-à-dire supérieure ou inférieure à 35 % mol, la quantité de peroxyde d'hydrogène devra être maintenu. En l'espèce, la solution de peroxyde d'hydrogène à 35 % mol est choisie car elle est facilement accessible dans le commerce. Typiquement, pour un fût 1 de 225 I, il a été observé qu'un volume compris entre 250 ml et 500 ml, c'est-à-dire notamment 250 ml, 260 ml, 270 ml, 280 ml, 290 ml, 300 ml, 310 ml, 320 ml, 330 ml, 340 ml, 350 ml, 360 ml, 370 ml, 380 ml, 390 ml, 400 ml, 410 ml, 420 ml, 430 ml, 440 ml, 450 ml, 460 ml, 470 ml, 480 ml, 490 ml et 500 ml, d'une solution de peroxyde d'hydrogène à 35 % mol donne satisfaction.

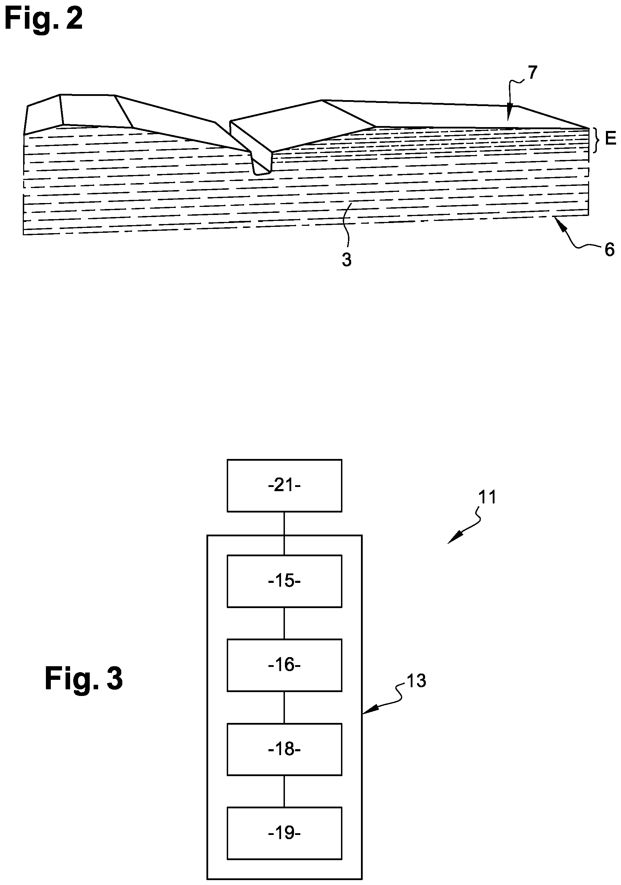

L'étape 13 de désinfection comporte en outre, préférentiellement, une phase 16 d'introduction de vapeur d'eau dans le fût 1 après la phase 15 d'introduction de peroxyde d'hydrogène à l'intérieur du fût 1. Le but de l'utilisation de la vapeur d'eau est d'améliorer la pénétration et l'efficacité de l'aseptisation dans le bois du fût 1. Concrètement, la phase 16 d'introduction de vapeur d'eau chauffe et ramollit les fibres du bois pour ouvrir suffisamment les pores du bois afin de faire pénétrer le peroxyde d'hydrogène dans le bois selon une épaisseur suffisante à enlever tout résidu qui y est incrusté, typiquement selon une épaisseur au moins égale celle E illustrée à la figure 2 comportant des résidus d'un élevage. En outre, la phase 16 d'introduction de vapeur d'eau permet d'amener le peroxyde d'hydrogène à une température où son efficacité d'aseptisation est optimale.

La phase 16 peut être obtenue par le montage d'une bonde dans le trou de bonde du fût 1 qui comporte une conduite d'arrivée de vapeur vers l'intérieur du fût 1 raccordé à un bouilleur permettant de mettre sous pression et de réchauffer l'intérieur du fût 1. Le bouilleur est préférentiellement paramétré afin de créer une vapeur d'eau humide, c'est-à-dire que l'eau sous forme de gaz est mélangée avec de l'eau sous forme liquide. Par conséquent, la phase 16 d'introduction peut injecter une vapeur d'eau comportant une proportion comprise entre 0 % et 40 % d'eau sous forme liquide en poids total de la vapeur d'eau humide injectée, le reste étant de l'eau vaporisée, c'est-à-dire sous forme de gaz.

La durée de la phase 16 est comprise entre deux et huit minutes en fonction de la température de réchauffement souhaité et de la puissance du bouilleur. Il a été observé que le peroxyde d'hydrogène était le plus efficace entre soixante degrés Celsius et quatre-vingts degrés Celsius, c'est-à-dire notamment 60°C, 61°C, 62°C, 63°C, 64°C, 65°C, 66°C, 67°C, 68°C, 69°C, 70°C, 71 °C, 72°C, 73°C, 74°C, 75°C, 76°C, 77°C, 78°C, 79°C et 80°C. De fait, préférentiellement, la phase 16 est utilisée pour amener le peroxyde d'hydrogène à au moins soixante degrés Celsius en évitant préférentiellement de dépasser quatre-vingts degrés Celsius. À titre d'exemple, un temps de six minutes avec une génération de vapeur de 40 kg·h⁻¹ a donné satisfaction. En outre, il a été observé qu'inverser les phases 15 et 16, c'est-à-dire réaliser la phase 16 d'introduction de vapeur puis la phase 15 d'introduction de peroxyde d'hydrogène à l'intérieur du fût, ne permet pas d'obtenir un aussi bon effet que mettre en oeuvre la phase 15 suivie de la phase 16 notamment au niveau de l'efficacité du retrait des résidus dans le bois tels que les pigments de vin rouge.

Selon une variante, un compresseur est monté entre le bouilleur et la conduite d'arrivée afin de réaliser la phase 16 sous une pression plus élevée comme par exemple vingt bars. Bien entendu, la pression maximale sera adaptée en fonction du fût 1 à nettoyer afin de ne pas exercer un pression interne susceptible d'endommager le fût 1. Le but de cette variante est de combiner la chaleur et la pression pour rendre plus efficace l'étape 13 de désinfection. Empiriquement, il a été trouvé que cette variante permet de diminuer le temps nécessaire à la phase 16 pour une même efficacité que sans l'utilisation de surpression.

Préférentiellement, l'étape 13 de désinfection comporte également une phase 18 de fermeture du fût 1 après la phase 16 d'introduction de vapeur d'eau dans le fût 1 afin de laisser le peroxyde d'hydrogène agir sur les composants du bois du fût 1 jusqu'à ce que la température du fût 1 redescende, préférentiellement naturellement, jusqu'à la température ambiante, c'est-à-dire préférentiellement sans refroidissement autre que les échanges avec l'air ambiant.

Concrètement, la phase 18 de fermeture peut être obtenue par fermeture des conduites d'arrivée ou par le remplacement de la bonde comportant ces conduites par une bonde 5 illustrée à la figure 1. On comprend que cette phase 18 de fermeture permet notamment de faire diminuer progressivement la température du volume intérieur du fût 1 fermé pour créer une dépression interne et refermer progressivement les pores du bois apte à améliorer l'évacuation de tout résidu infiltré dans les fibres du bois sans que les résidus extraits ne puissent s'y incruster à nouveau. Ainsi, la dépression va favoriser un flux d'air au travers des pores du bois de la face externe 6 vers la face interne 7 et la contraction des pores du bois va mécaniquement expulser tout résidu présent dans les pores vers le volume intérieur du fût 1.

De plus, la phase de fermeture 18 du fût est maintenue pendant au moins une durée prédéterminée préférentiellement dépendante de la température à laquelle le fût 1 a été chauffé pendant la phase 16 d'introduction de la vapeur d'eau. Typiquement, plus la température est élevée, moins la phase 18 de fermeture pourrait être longue car le peroxyde d'hydrogène agira plus rapidement mais plus il sera nécessaire d'attendre s'il est souhaité que la température du fût 1 redescende naturellement jusqu'à la température ambiante. Ainsi, plus le peroxyde d'hydrogène est chaud (typiquement entre soixante degrés Celsius et quatre-vingts degrés Celsius) et est maintenu chaud pendant la phase 18 de fermeture, plus son action oxydante est forte. La durée de la phase 18 de fermeture peut être comprise entre une heure et quatre heures suivant le fût 1 utilisé, c'est-à-dire notamment 1 heure, 1,5 heure, 2 heures, 2,5 heures, 3 heures, 3,5 heures et 4 heures. Il a été constaté qu'une durée de phase 18 de fermeture de deux heures pour un fût 1 de 225 litres donnait satisfaction.

Si la différence de température par rapport à la température ambiante est très importante, il pourra être nécessaire de prévoir un dispositif de compensation de pression comme une soupape, par exemple dans la bonde, pour éviter que la dépression à l'intérieur du fût 1, créée par le refroidissement, induise la détérioration des fonds 2 du fût 1.

Lors de l'étape de désinfection, le fût 1 peut être déplacé sur lui-même pour améliorer l'homogénéisation de l'aseptisation de l'intérieur du fût 1. Typiquement, le déplacement sur lui-même peut être envisagé après la phase d'introduction de peroxyde d'hydrogène à l'intérieur du fût et/ou lors de la phase de fermeture du fût. Un tel déplacement peut consister en un remuage du fût 1 manuellement ou à l'aide d'un mécanisme de mise en rotation du fût 1 dont l'axe de rotation est déplaçable pour induire un mouvement de remuage complexe automatique.

Préférentiellement, l'étape 13 de désinfection comporte enfin une phase finale 19 de lavage du fût 1 afin de rincer le bois de l'intérieur du fût 1. Cette phase peut également être utilisée pour détartrer l'intérieur du fût 1. Ainsi, la phase 19 de lavage peut, à titre nullement limitatif, comporter un premier cycle de circulation d'eau chaude dans le fût 1, c'est-à-dire l'introduction dans le fût 1 par une conduite d'entrée passant par le trou de bonde avec la libre possibilité de s'échapper du fût 1 par une sortie (qui peut être le trou de bonde orienté vers le bas), comme par exemple entre quarante degrés Celsius et quatre-vingts degrés Celsius pendant une durée comprise entre une minute et dix minutes, suivie d'un deuxième cycle de circulation d'eau froide ozonée, c'est-à-dire comportant de l'ozone (O₃), dans le fût 1, comme par exemple à température du réseau de distribution d'eau, typiquement entre quinze degrés Celsius et vingt degrés Celsius pendant une durée comprise entre une minute et dix minutes. Un cycle optionnel de circulation de vapeur d'eau peut être prévu pendant une durée comprise entre une minute et dix minutes entre les premier et deuxième cycles.

Le demandeur a fait réaliser des mesures sur des fûts 1 ayant été utilisés pour notamment des élevages de vin rouge et ayant bénéficiés de la mise en oeuvre du procédé 11 de nettoyage selon l'invention. Les tests ont permis de vérifier que le pouvoir oxydant du peroxyde d'hydrogène permet de diminuer à une quantité négligeable voire indétectable tout élément à base de soufre utilisé pendant l'élevage tel que, par exemple, du soufre ou des oxydes de soufre.

Il a pu être également contrôlé que la surface interne 7 du fût 1 ne comporte plus de peroxyde d'hydrogène ni qu'elle soit plus sensible à l'oxydation qu'un bois neuf. En outre, toute coloration du bois, occasionnée lors de l'élevage, est retirée permettant de retrouver un aspect neutre du bois sur tout le fût 1 notamment sans dégradation des fibres du bois.

Enfin, la surface interne 7 du fût 1 est, avantageusement selon l'invention, entièrement désinfectée en éliminant notamment toute acidité volatile ou de surface comme de l'acétate d'éthyle. Le fût 1 est donc rendu totalement neutre par rapport à l'élevage de la boisson alcoolique pour lequel il a pu être utilisé ce qui lui permet de pouvoir être réutilisé notamment pour un nouvel élevage de boisson alcoolique dans des conditions similaires à un fût 1 neuf. Il a aussi pu être mesuré que la teneur en ellagitanins est plus basse qu'un fût neuf ce qui permet de rendre le fût 1 nettoyé par le procédé 11 selon l'invention plus neutre qu'un fût 1 neuf.

L'invention n'est pas limitée aux modes de réalisation et variantes présentés et d'autres modes de réalisation et variantes apparaîtront clairement à l'homme du métier. Ainsi, les modes de réalisation et variantes sont combinables entre eux sans sortir du cadre de l'invention. À titre nullement limitatif, il peut être envisagé de réaliser un traitement de chauffe de la surface interne 7 du fût 1 permettant de personnaliser les profils aromatiques du fût 1 après la mise en oeuvre du procédé 11 selon l'invention sans difficulté notable par rapport à un fût 1 neuf.

## Revendications

1. Procédé (11) de nettoyage d'un fût (1) en bois **caractérisé en ce qu'**il comporte une étape (13) de désinfection de l'intérieur (7) du fût (1) comprenant au moins une phase (15) d'introduction de peroxyde d'hydrogène à l'intérieur du fût (1) et une phase (16) d'introduction de vapeur d'1eau dans le fût (1) après la phase (15) d'introduction de peroxyde d'hydrogène à l'intérieur du fût (1) afin d'aseptiser le bois du fût (1) en profondeur et de retirer tout résidu infiltré dans les fibres du bois tel que des pigments.

2. Procédé (11) de nettoyage d'un fût (1) en bois **caractérisé en ce qu'**il comporte une étape (13) de désinfection de l'intérieur (7) du fût (1) comprenant au moins une phase (15) d'introduction de peroxyde d'hydrogène à l'intérieur du fût (1) et une phase (16) d'introduction de vapeur d'eau dans le fût (1) avant la phase (15) d'introduction de peroxyde d'hydrogène à l'intérieur du fût (1) afin d'aseptiser le bois du fût (1) en profondeur et de retirer tout résidu infiltré dans les fibres du bois tel que des pigments.

3. Procédé (11) selon la revendication 1, dans lequel, lors de la phase (15) d'introduction de peroxyde d'hydrogène à l'intérieur du fût (1), une solution de peroxyde d'hydrogène à 35 % mol est insérée dans le fût (1) selon une proportion de 0,5 ml à 3,5 ml par litre de volume intérieur de fût (1) et, préférentiellement, selon une proportion de 1,0 ml à 2,3 ml par litre de volume intérieur de fût (1).

4. Procédé (11) selon l'une des revendications 1 ou 3, dans lequel la phase (16) d'introduction de vapeur d'eau dans le fût (1) est utilisée pour amener le peroxyde d'hydrogène à l'intérieur du fût (1) à une température comprise entre 60°C et 80°C.

5. Procédé (11) selon l'une des revendications 1 ou 3 à 4, dans lequel l'étape (13) de désinfection comporte une phase (18) de fermeture du fût (1) après la phase (16) d'introduction de vapeur d'eau dans le fût (1) pour, en refroidissant, créer une dépression interne et refermer progressivement les pores du bois afin d'améliorer l'évacuation de tout résidu infiltré dans les fibres du bois.

6. Procédé (11) selon la revendication précédente, dans lequel la durée de la phase (18) de fermeture est comprise entre une heure et quatre heures.

7. Procédé (11) selon la revendication 5 ou 6, dans lequel, lors de l'étape (13) de désinfection, le fût (1) est déplacé sur lui-même pour améliorer l'homogénéisation de l'aseptisation de l'intérieur du fût (1).

8. Procédé (11) selon l'une des revendications 1 ou 3 à 7, dans lequel l'étape (13) de désinfection comporte une phase (19) finale de lavage de l'intérieur du fût (1).

9. Procédé (11) selon l'une des revendications 1 ou 3 à 8, comportant une étape (21) de prélavage de l'intérieur du fût (1) avant l'étape (13) de désinfection.

## Patentansprüche

1. Verfahren (11) zur Reinigung eines Fasses (1) aus Holz, **dadurch gekennzeichnet, dass** es einen Schritt (13) des Desinfizierens des Inneren (7) des Fasses (1) aufweist, wenigstens umfassend eine Phase (15) des Einleitens von Wasserstoffperoxid in das Innere des Fasses (1) und eine Phase (16) des Einleitens von Wasserdampf in das Fass (1) nach der Phase (15) des Einleitens von Wasserstoffperoxid in das Innere des Fasses (1), um das Holz des Fasses (1) gründlich zu aseptisieren und jegliche in die Fasern des Holzes eingedrungenen Rückstände wie Pigmente zu entziehen.

2. Verfahren (11) zur Reinigung eines Fasses (1) aus Holz, **dadurch gekennzeichnet, dass** es einen Schritt (13) des Desinfizierens des Inneren (7) des Fasses (1) aufweist, wenigstens umfassend eine Phase (15) des Einleitens von Wasserstoffperoxid in das Innere des Fasses (1) und eine Phase (16) des Einleitens von Wasserdampf in das Fass (1) vor der Phase (15) des Einleitens von Wasserstoffperoxid in das Innere des Fasses (1), um das Holz des Fasses (1) gründlich zu aseptisieren und sämtliche in die Fasern des Holzes eingedrungenen Rückstände wie Pigmente zu entziehen.

3. Verfahren (11) nach Anspruch 1, wobei, während der Phase (15) des Einleitens von Wasserstoffperoxid in das Innere des Fasses (1), eine Wasserstoffperoxid-Lösung mit 35 Mol-% in das Fass (1) gemäß einem Anteil von 0,5 ml bis 3,5 ml pro Liter Innenvolumen des Fasses (1) und, vorzugsweise, gemäß einem Anteil von 1,0 ml bis 2,3 ml pro Liter Innenvolumen des Fasses (1) eingebracht wird.

4. Verfahren (1) nach einem der Ansprüche 1 oder 3, wobei die Phase (16) des Einleitens von Wasserdampf in das Fass (1) verwendet wird, um das Wasserstoffperoxid im Inneren des Fasses (1) auf eine Temperatur zwischen 60 °C und 80 °C zu bringen.

5. Verfahren (1) nach einem der Ansprüche 1 oder 3 bis 4, wobei der Schritt (13) des Desinfizierens eine Phase (18) des Verschlusses des Fasses (1) nach der Phase (16) des Einleitens von Wasserdampf in das Fass (1) aufweist, um, durch Kühlen, einen Innenunterdruck zu erzeugen und die Poren des Holzes progressiv zu schließen, um das Ableiten jeglicher in die Fasern des Holzes eingedrungenen Rückstände zu verbessern.

6. Verfahren (1) nach dem vorhergehenden Anspruch, wobei die Dauer der Phase (18) des Verschlusses des Fasses (1) zwischen einer Stunde und vier Stunden ist.

7. Verfahren (1) nach Anspruch 5 oder 6, wobei, während des Schrittes (13) des Desinfizierens, das Fass (1) selbst bewegt wird, um die Homogenisierung der Aseptisierung des Inneren des Fasses (1) zu verbessern.

8. Verfahren (1) nach einem der Ansprüche 1 oder 3 bis 7, wobei der Schritt (13) des Desinfizierens eine abschließende Phase (19) des Waschens des Inneren des Fasses (1) aufweist.

9. Verfahren (1) nach einem der Ansprüche 1 oder 3 bis 8, aufweisend einen Schritt (21) des Vorwaschens des Inneren des Fasses (1) vor dem Schritt (13) des Desinfizierens.

## Claims

1. Method (11) for cleaning a wooden barrel (1) **characterised in that** it comprises a step (13) of disinfecting the inside (7) of the barrel (1) including at least one phase (15) of introducing hydrogen peroxide inside the barrel (1) and a phase (16) of introducing steam in the barrel (1) after the phase (15) of introducing hydrogen peroxide inside the barrel (1) in order to sterilise the wood of the barrel (1) in depth and remove any residue infiltrated in the wood fibres such as pigments.

2. Method (11) for cleaning a wooden barrel (1) **characterised in that** it comprises a step (13) of disinfecting the inside (7) of the barrel (1) including at least one phase (15) of introducing hydrogen peroxide inside the barrel (1) and a phase (16) of introducing steam in the barrel (1) before the phase (15) of introducing hydrogen peroxide inside the barrel (1) in order to sterilise the wood of the barrel (1) in depth and remove any residue infiltrated in the wood fibres such as pigments.

3. Method (11) according to claim 1, wherein, during the phase (15) of introducing hydrogen peroxide inside the barrel (1), a 35 % mol solution of hydrogen peroxide is inserted in the barrel (1) in a proportion of 0.5 mL to 3.5 mL per litre of inner volume of barrel (1) and, preferably, in a proportion of 1.0 mL to 2.3 mL per litre of inner volume of barrel (1).

4. Method (11) according to one of claims 1 or 3, wherein the phase (16) of introducing steam in the barrel (1) is used to raise the temperature of the hydrogen peroxide inside the barrel (1) to between 60 °C and 80 °C.

5. Method (11) according to one of claims 1 or 3 to 4, wherein the step (13) of disinfecting comprises a phase (18) of closing the barrel (1) after the phase (16) of introducing steam in the barrel (1) in order, on cooling, to create an internal depression and progressively close the wood pores to improve the evacuation of any residue infiltrated in the wood fibres.

6. Method (11) according to the preceding claim, wherein the duration of the phase (18) of closing is between 1 hour and 4 hours.

7. Method (11) according to claim 5 or 6, wherein, during the step (13) of disinfecting, the barrel (1) is displaced about itself so that the sterilisation of the inside of the barrel (1) is more homogeneous.

8. Method (11) according to one of claims 1 or 3 to 7, wherein the step (13) of disinfecting comprises a final phase (19) of washing the inside of the barrel (1).

9. Method (11) according to one of claims 1 or 3 to 8, comprising a step (21) of prewashing the inside of the barrel (1) before the step (13) of disinfecting.
